(19) Europäisches Patentamt
European Patent Office
Office européen des brevets

(11) EP 2 602 337 A1

(12) **EUROPEAN PATENT APPLICATION**
published in accordance with Art. 153(4) EPC

(43) Date of publication:
**12.06.2013 Bulletin 2013/24**

(21) Application number: **10852899.3**

(22) Date of filing: **11.06.2010**

(51) Int Cl.:
***C22C 19/07*** *(2006.01)* ***A61F 2/30*** *(2006.01)*

(86) International application number:
**PCT/JP2010/059946**

(87) International publication number:
**WO 2011/155063 (15.12.2011 Gazette 2011/50)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(71) Applicant: **Kyocera Medical Corporation**
**Osaka-shi, Osaka 532-0003 (JP)**

(72) Inventors:
- **ISHIMIZU, Keita**
**Osaka-shi**
**Osaka 532-0003 (JP)**
- **NANBA, Shigenobu**
**Kobe**
**Hyogo 651-2271 (JP)**

(74) Representative: **Müller-Boré & Partner**
**Patentanwälte**
**Grafinger Straße 2**
**81671 München (DE)**

# (54) CAST BASE FOR BIOMEDICAL USE FORMED OF COBALT/CHROMIUM-BASED ALLOY AND HAVING EXCELLENT DIFFUSION HARDENING TREATABILITY, SLIDING ALLOY MEMBER FOR BIOMEDICAL USE AND ARTIFICIAL JOINT

(57) A cast base for biomedical use, which is formed of a cobalt-chromium based alloy and has excellent diffusion hardening treatability, characterized by containing 0.1 mass% or greater of nitrogen (N) and having a volume fraction of an fcc (face centered cubic lattice) phase in the metallic structure thereof of 50% or greater. Even in the case of subjecting the aforesaid cobalt-chromium based alloy cast base in the as-cast state, the structure of which is liable to be non-uniform, to a diffusion hardening treatment, it is possible to obtain a sliding alloy member for biomedical use, wherein a uniform hardened layer has been formed, by the diffusion hardening treatment. Thus, a sliding alloy member for biomedical use and so on, which can stably and continuously exhibit high strength, excellent abrasion resistance and, moreover, excellent corrosion resistance, can be provided.

FIG.9

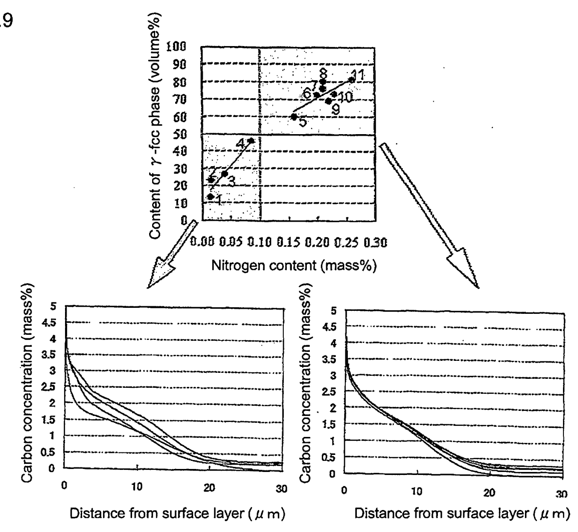


EP 2 602 337 A1

**Description**

TECHNICAL FIELD

The present invention relates to a cast base for biomedical use formed of a cobalt-chromium based alloy and having excellent diffusion hardening treatability, a sliding alloy member for biomedical use, and an artificial joint. The present invention relates to the cast base for biomedical use formed of a cobalt-chromium based alloy which can be hardened evenly in the base surface by diffusion hardening treatment, for example, carburizing, nitriding, etc. (in the present invention, such a characteristic is referred to as "having excellent diffusion hardening treatability"), the sliding alloy member for biomedical use, which is obtained by carrying out diffusion hardening treatment such as carburizing, nitriding, etc. for the cast base formed of the cobalt-chromium based alloy and which stably shows excellent wear resistance, and the artificial joint using the sliding alloy member for biomedical use.

BACKGROUND ART

Being excellent in biocompatibility, strength, wear resistance, and corrosion resistance among metal materials for biomedical use, a cobalt-chromium based alloy has been used for a long time for a sliding member of an artificial joint such as an artificial hip joint or the like; an implant member, etc. For example, Patent Document 1 discloses a cobalt-chromium-molybdenum alloy with a defined component composition. Patent Document 2 discloses a method for producing a Co based alloy with a defined component composition and $\gamma$-phase amount, aiming improvement of plastic workability.

In the case such a cobalt-chromium based alloy is used for particularly a sliding member for biomedical use, since the surface is easy to be worn, carburizing, nitriding, and the like are commonly carried out for surface hardening treatment. For example, Patent Document 3 and Patent Document 4 disclose methods for carrying out carburizing for cobalt-chromium based alloy materials and characteristics (improvement of the surface hardness and wear resistance without deteriorating corrosion resistance,etc.) provided accordingly.

Patent Document 5 discloses execution of diffusion hardening treatment for metal materials including a Co-Cr-Mo alloy and describes concretely that the alloy surface is strengthened and hardened by an internal oxidation method, an internal nitriding method, an additional and interstitial diffusion strengthening method using nitrogen, oxygen, or carbon.

PRIOR ART DOCUMENTS

PATENT DOCUMENTS

Patent Document 1: JP-A-54-10224
Patent Document 2: JP-A-2008-111177
Patent Document 3: JP-A-2005-524772
Patent Document 4: JP-A-2007-277710
Patent Document 5: JP-B1- 3471041

SUMMERY OF THE INVENTION

PROBLEMS TO BE SOLVED BY THE INVENTION

However, a base of a cobalt-chromium based alloy has a microstructure (hereinafter, sometimes referred to as metallic structure or simply structure) considerably fluctuated by the production method, added element amounts, heat treatment, etc. and as a result, the characteristics are considerably changed. Especially, an as-cast base obtained by a typical base production method including melting a metal, pouring the melted metal in a die, and solidifying the melted metal has a structure in significantly uneven state (the state that deviation (segregation) of the element concentration is caused during the melted metal solidification process, hereinafter, the same).

As a method for converting the structure in the above-mentioned uneven state into in an even structure, there is a method for carrying out a high temperature and long duration heat treatment for an as-cast alloy. On the other hand, in recent years, as a typical combination of sliding members for an artificial joint, a cobalt-chromium based alloy sliding member and a cobalt-chromium based alloy sliding member are sometimes combined, and in such a combination, it has been known well that the wear resistance can be improved by increasing the carbon amount in the cobalt-chromium based alloy and dispersing much more carbides, which are compounds consisting of carbon and metals, in the alloy.

Accordingly, heat treatment at such a high temperature as to remove the carbides is often not carried out.

As described above, in order to improve the wear resistance, high temperature heat treatment tends not to be carried out. But for a base which is left as it is without being subjected to the heat treatment at high temperature, and therefore has uneven structure, diffusion hardening treatment such as carburizing, nitriding is to be carried out. However, if the diffusion hardening treatment is carried out for the base having an uneven structure, in a single treatment plane, hardening is sometimes locally deficient and solid solution elements (e.g., carbon in the carburizing) exhibit element concentration distribution with no reproducibility in the thickness direction of the hardened layer and it leads to a problem that the wear resistance tends to vary.

In view of the above state of the art, it is an object of the present invention to provide a cast base for biomedical use formed of a cobalt-chromium based alloy for which hardening of the base surface can be carried out sufficiently evenly in the case that diffusion hardening treatment is carried out, a sliding alloy member for biomedical use which is obtained by the above-mentioned diffusion hardening treatment for the cast base formed of the cobalt-chromium based alloy and which stably exhibits excellent wear resistance, and an artificial joint with high reliability obtained by using the sliding alloy member for biomedical use.

## SOLUTIONS TO THE PROBLEMS

A cast base for biomedical use formed of a cobalt-chromium based alloy of the present invention is a cast base for biomedical use produced from a cobalt-chromium based alloy and has a feature of containing 0.1 mass% or more of nitrogen (N) and having volume fraction of an fcc (face centered cubic lattice) phase in the metallic structure of 50% or higher (% in the metallic structure is volume%, hereinafter, the same).

The N amount of the cobalt-chromium based alloy is preferably 0.25 mass% in the upper limit and the contents of elements other than N are preferably within ranges standardized in ASTM F75-07.

The cast base formed of the cobalt-chromium based alloy of the present invention has an average crystal grain size in the metallic structure of 1000 μm or greater.

The present invention also includes a sliding alloy member for biomedical use obtained by diffusion hardening treatment (e.g. carburizing) of the cast base formed of the cobalt-chromium based alloy in as-cast state. The present invention also includes an artificial joint composed of 2 sliding members forming a sliding face, which are cobalt-chromium based alloy sliding members, and has a feature that at least one of the cobalt-chromium based alloy sliding members is the above-mentioned sliding alloy member for biomedical use.

## EFFECTS OF THE INVENTION

According to the invention, even if a cast base formed of a cobalt-chromium based alloy in as-cast state in which the structure tends to be uneven is subjected to diffusion hardening treatment, a sliding alloy member for biomedical use having a more evenly hardened layer by the diffusion hardening treatment can be obtained. As a result, the present invention can provide a sliding alloy member for biomedical use which can stably exhibit high strength, excellent wear resistance and excellent corrosion resistance, and an artificial joint or the like with high reliability by using the sliding alloy member for biomedical use.

## BRIEF DESCRIPTION OF THE DRAWINGS

[Fig. 1] Fig. 1 is a Vickers indentation photograph of a surface of a base after carburizing (No. 2 in examples).

[Fig. 2] Fig. 2 is a Vickers indentation photograph of a surface of a base after carburizing (No. 7 in examples).

[Fig. 3] Fig. 3 is a photograph showing crystal orientation analysis results of a base before carburizing (No. 2 in examples) by an electron back scattering pattern (EBSP) method.

[Fig. 4] Fig. 4 is a photograph showing crystal orientation analysis results of a base before carburizing (No. 7 in examples) by an electron back scattering pattern (EBSP) method.

[Fig. 5] Fig. 5 is a Vickers indentation photograph of a surface of a base before carburizing (No. 2 and No. 7 in examples).

[Fig. 6] Fig. 6 is a graph showing a relationship between a nitrogen content in a base and a volume fraction of an fcc phase.

[Fig. 7] Fig. 7 is a graph showing a relationship between a carbon content in a base and a volume fraction of an fcc phase.

[Fig. 8] Fig. 8 is a representative macro-structure photograph of a cast base formed of a cobalt-chromium based alloy of a present invention.

[Fig. 9] Fig. 9 is a view showing a carbon concentration distribution of a carburized layer separately for examples in a region A and examples in a region B.

MODE FOR CARRYING OUT THE INVENTION

Inventors of the present invention have made extensive investigations to develop a cast base for biomedical use formed of a cobalt-chromium based alloy for which an evenly hardened layer free from local hardening deficiency can be formed, particularly, by diffusion hardening treatment. As a result, the inventors of the present invention have found that if the metallic structure of the cast base formed of a cobalt-chromium based alloy is made to be a structure in which a crystal structure contains a prescribed volume of fcc (face centered cubic lattice) phase (also referred to as fcc phase or $\gamma$-phase), the aim can be accomplished. Hereinafter, the present invention will be described in detail. The case of carrying out carburizing as a representative diffusion hardening treatment is described below, the present invention is not limited to that, and, as described below, nitriding, boronizing, oxidizing, and the like are also within the technical scope of the present invention.

The inventors of the present invention considered at first the local hardening deficiency could be attributed to the metallic structure, and produced cast bases formed of cobalt-chromium based alloys (in as-cast state) with various kinds of metallic structures, carried out carburizing according to the methods described in Examples as follows, and subsequently, measured the Vickers hardness at a plurality of points (hereinafter, sometimes referred to simply as hardness) in a single plane of each carburized material. The measurement was carried out at 10 arbitrary points for each specimen using a load of 50 gf with a Vickers hardness measurement apparatus.

As a result, it was found that there are specimens with uneven hardness in a single plane and specimens with scarce dispersion of hardness. The inventors of the present invention, therefore, investigated the state of non-uniform hardness and causes of the occurrence for the specimen (No. 2 of Example described below) with uneven hardness in a single plane (hereinafter, such non-uniform hardness in a single plane is sometimes referred to as "non-uniform hardness") and for the specimen (No. 7 of Example described below) with little unevenness of hardness.

Regarding the specimen (No. 2) having non-uniform hardness, the Vickers hardness values measured at a plurality of points in a single plane were classified into relatively high hardness and relatively low hardness and consequently, as shown in Table 1, it was understood that the relatively high hardness was measured around precipitates (carbides) existing after casting and on the other hand, the relatively low hardness was measured in regions apart from the precipitates. It can be confirmed also based on the difference of the size of Vickers indentation in the surrounding of the precipitates and in the other regions in a Vickers indentation photograph of No. 2 (optical microscopic photograph, as the indentation is smaller, it shows the hardness is harder) shown in Fig. 1. The result of classification of Vickers hardness measured for No. 7 in the surrounding of the precipitates (carbides) and the other regions (regions apart from the precipitates) is also shown in Table 1. The Vickers indentation photograph of No. 7 is shown in Fig. 2, and from Table 1 and Fig. 2, it can be understood that the hardness is almost constant regardless of the measurement points for No. 7.

[Table 1]

| | Vickers hardness of No. 2 (Hv) | | Vickers hardness of No. 7 (Hv) | |
|---|---|---|---|---|
| | Surrounding regions of precipitates | Regions apart from precipitates | Surrounding regions of precipitates | Regions apart from precipitates |
| 1 | 1072 | 666 | 1027 | 1145 |
| 2 | 1145 | 733 | 1145 | 1006 |
| 3 | 1115 | 773 | 1095 | 1038 |
| 4 | 1199 | 707 | 1006 | 996 |
| 5 | 1107 | 695 | 1061 | 1072 |
| Average value | 1128 | 715 | 1067 | 1051 |

To clarify the cause of occurrence of non-uniform hardness, the metallic structure of the above-mentioned No. 2 was investigated. Concretely, the crystal orientation analysis of the base of No. 2 before carburizing was carried out by an electron back scattering pattern (EBSP) method. The results are shown in Fig. 3. Fig. 3 (a) shows the blue regions as the regions of fcc phase; and Fig. 3 (b) shows the red regions as the regions of hcp phase (lattice of hexagonal closest packing, also referred to as $\varepsilon$-phase). From Fig. 3, it is understood that the surroundings of the precipitates of various shapes are regions of fcc phase and the regions apart from the precipitates are the hcp phase.

From the structure shown in Fig. 3 and the Vickers indentation photograph shown in Fig. 1, it is understood that in the structure before the carburizing, the surroundings of precipitates, i.e.the fcc phase, have high hardness after the carburizing, and the regions apart from the precipitates, i.e.the hcp phase, have hardness lower than that of the surroundings of the precipitates after the carburizing.

Fig. 4 shows the result of crystal orientation analysis by EBSP for No. 7 having the Vickers hardness (Vickers indentation) almost constant regardless of the measurement points. In Fig. 4, the green, blue-green, and blue portions show fcc phase regions. From Fig. 4, it can be understood that the structure before the carburizing is almost completely occupied with the fcc phase in No. 7.

Whether the non-uniform hardness is caused by carburizing or not (how the non-uniform hardness is before carburizing) was investigated. That is, as described above, Vickers hardness were measured and photographs of Vickers indentation were taken for the bases of No. 2 and No. 7 before carburizing. The results are shown in Table 2 and Fig. 5.

[Table 2]

| | Vickers hardness of No. 2 (Hv) | | Vickers hardness of No. 7 (Hv) | |
|---|---|---|---|---|
| | Surrounding regions of precipitates | Regions apart from precipitates | Surroundirig regions of precipitates | Regions apart from precipitates |
| 1 | 336 | 341 | 321 | 334 |
| 2 | 341 | 355 | 347 | 336 |
| 3 | 327 | 341 | 327 | 358 |
| 4 | 358 | 360 | 349 | 358 |
| 5 | 334 | 358 | 394 | 331 |
| Average value | 339 | 351 | 348 | 343 |

From Table 2 and Fig. 5, before the carburizing, it was found that both No. 2 and No. 7 had hardness almost even in a single plane and the non-uniform hardness was generated by executing carburizing.

From these results, it was found that if the ratio of the hcp phase in the structure of a base was significantly higher than that of the fcc phase before carburizing, the non-uniform hardness became considerable since the hardness of the hcp phase regions was lowered after the carburizing, and accordingly, if the ratio of the fcc phase in the structure of the base before carburizing was increased, the regions with high hardness could be reliably formed by carrying out the carburizing and the non-uniform hardness could be suppressed.

The inventors of the present invention made investigations to determine how far extent the fcc phase turned to be hard after carburizing should be attained in the structure of a base before the carburizing. Concretely, various kinds of cast base formed of a cobalt-chromium based alloy with different volume fractions of the fcc phase were produced by changing the component compositions as shown in Table 3 of Examples described below, and the non-uniform hardness was investigated for the respective bases. As a result, it was found that if the volume fraction of the fcc phase in the structure of the base before the carburizing was controlled to be 50% or higher, the Vickers hardness became almost constant in a single plane after carburizing regardless of existence of carbides or the existence positions, and a uniform hardened layer free from local hardness deficiency could be formed.

Since the in-plane uniformity of the hardness is heightened more as the ratio of the fcc phase is increased more, the volume fraction of the fcc phase is preferably 60% or higher and more preferably 70% or higher. On the other hand, in consideration that the upper limit of the N content is standardized to be 0.25 mass% in ASTM F75-07 in terms of the balance between the strength and ductility as described below, the upper limit of the volume fraction of the fcc phase is around 85%.

In the present invention, the ratio of the fcc phase in the structure of the base is sufficient to be 50% or higher as described before, and the present invention may include the case that the base contains other structures which remain inevitably during the production process of the base. Concretely, the above-mentioned hcp phase and precipitates of carbides, nitrides, carbonitrides, intermetallic compounds, etc. may be contained.

Next, the inventors of the present invention made investigations on a practical method for obtaining the structure in which 50% or higher was occupied by the fcc phase. As described above, the structure is made uniform by carrying out high temperature heat treatment, but it also results in an adverse consequence such that the carbides are removed and the wear resistance of the base is lowered. Therefore, paying attention to the component composition rather than the production condition, the inventors of the present invention made investigations.

The inventors of the present invention produced cast bases formed of cobalt-chromium based alloys with various component compositions as shown in Table 3 in Examples described below, measured the ratio of the fcc phase in the metallic structure of each base according to the method described in Examples described below, and investigated the relation of the respective component elements and the ratio of the fcc phase. As a result, it was found that the content of N among various elements, had correlation with the ratio of the fcc phase.

Fig. 6 is a graph obtained from the N amount (nitrogen content) and the volume fraction of the fcc phase in the above-mentioned cast base formed of a cobalt-chromium based alloy (the reference numeral in the graph shows the No. in Table 3 of Examples described below, and same for Fig. 7 and Fig. 9, as described below). From this Fig. 6, it was found that the N amount and the volume fraction of the fcc phase in the above-mentioned base had correlation and if the N amount is low, the fcc phase was extremely decreased, and that addition of 0.1 mass% or higher of N stably guaranteed 50% or higher of the fcc phase.

For reference, the correlation of the content of C (carbon content) as another component besides N and the volume fraction of the fcc phase is shown in Fig. 7. From this Fig. 7, it was found clearly that there was no correlation between the C amount and the volume fraction of the fcc phase in the above-mentioned base.

The present invention can have a feature that even being kept as cast (in as-cast state), a cast base formed of a cobalt-chromium based alloy can have a structure in which the ratio of 2 kind phases, the fcc phase and the hcp phase, typical crystal structure composing the cast base formed of cobalt-chromium based alloy is controlled stably to be 50% or higher of the fcc phase by adjusting the N amount in the cast base formed of the cobalt-chromium based alloy to be 0.1 or higher, and as a result, a hardened layer with high reproducibility and uniform hardness can be formed when diffusion hardening treatment such as carburizing is carried out.

It is preferable to control the N amount to be 0.15 mass% or higher in order to increase the volume fluctuation of the fcc phase in the metallic phase, as described before, to preferably 60% or higher and to obtain a hardened layer with more uniform hardness by diffusion hardening treatment. It is more preferable to control the N amount to be 0.20 mass% or higher in order to increase the volume fluctuation of the fcc phase to more preferably 70% or higher and to obtain a hardened layer with still more uniform hardness by diffusion hardening treatment.

Patent Document 1 and Patent Document 2 have description that the N amount is defined; however the effect is limited to the improvement of strength, ductility, and corrosion resistance, or to the improvement of plastic formability, and do not describe or imply the in-plane evenness of hardness by diffusion hardening treatment.

In the present invention, regarding the component composition, it is required to control particularly the N amount to be a prescribed amount of higher, as described above, the upper limit of the N amount and the contents of other elements may be within ranges for conventionally known cobalt-chromium based alloys for biomedical use. For example, as standardized in ASTM F75-07, the upper limit of the N amount may be controlled to be 0.25 mass% and the contents of other elements may be determined together. Concretely, examples may be those which contain Cr: 27.00 to 30.00 mass%, Mo: 5.00 to 7.00 mass%, Ni: 0.50 mass% or less, Fe: 0.75 mass% or less, C: 0.35 mass% or less, N: 0.1 to 0.25 mass%, Si: 1.00 mass% or less, Mn: 1.00 mass% or less, and residual Co and inevitable impurities. The lower limit of C amount is preferably 0.15 mass% in terms of formation of carbides in the base.

A method for producing the above-mentioned cast base formed of a cobalt-chromium based alloy is not particularly limited and may be, for example, melting a cobalt-chromium based alloy with controlled components, casting the melted alloy by near-net shape casting, that is, pouring the melted alloy in a die such as a bone head die for an artificial joint, and obtaining a base (in as-cast state). As described above, to increase the N amount in the cast base formed of a cobalt-chromium based alloy, nitrogen gas may be introduced at the time of melting, or nitrides such as $Cr_2N$, CrN, FeCrN, $Si_3N_4$, MnN, etc. may be added. After casting, the surface may be ground a little for removing the surface defects and roughness.

The cast base formed of a cobalt-chromium based alloy kept in as-cast state without heat treatment after forging is subjected to diffusion hardening treatment so that the carbides in the metallic structure are maintained without elimination and as a result, excellent wear resistance can be reliably attained. Heat treatment (particularly by heating at a temperature of 1000°C or higher) is carried out for the base, carbides are eliminated and therefore, it is not preferable.

A sliding alloy member for biomedical use composing an artificial joint or the like can be obtained by carrying out diffusion hardening treatment for the cast base formed of the cobalt-chromium based alloy in the as-cast state. The diffusion hardening treatment may be nitriding, boronizing, and oxidizing and the like beside the above-mentioned carburizing and any of these treatments can cause same effect as that of the carburizing treatment. The base (or the base subjected to the activation treatment as described below) may be treated at a commonly employed temperature by, for example, putting the base in treatment furnace and introducing a gas mixture containing a carbon source, a nitrogen source, and the like into the furnace.

For example, the carburizing may be carried out in the following condition. That is, the carburizing may be carried out by controlling the temperature (carburizing temperature) of the base to be 450 to 550°C. If the temperature is within the range, carbon forms a solid-solution in the surface of the base, but hardly forms chromium carbide and it is therefore preferable. If the carburizing temperature is lower than 450°C, solid-solution of carbon is not promoted and a solid-solution layer having a desirable surface hardness cannot be formed and therefore, it is not preferable. If it is higher than 550°C, formation of chromium carbide is promoted and therefore, it is not preferable.

As a carbon source for the carburizing, one or more kind compounds, e.g., CO, $CO_2$, $CH_4$, $C_2H_6$, $C_3H_8$, and $C_4H_{10}$ may be used. After diluted with an inert gas, a gas mixture of the above-mentioned carbon source and for example, $H_2$ may be introducing into the treatment furnace. $N_2$, Ar, and He may be used as the inert gas. The time of the carburizing may be controlled in accordance with the relation of the treatment temperature, and the thickness of the solid-solution layer and it is generally 1 to 50 hours and most commonly 10 to 35 hours.

Before the diffusion hardening treatment, activation treatment may be carried out to remove the passivation film formed on the base surface. Chromium in the base forms the passivation film by reaction with oxygen in air. The passivation film tends to inhibit penetration of the base surface with carbon when the carburizing is carried out. Accordingly, carburization is sufficiently carried out by removing the passivation film by the activation treatment. The activation treatment is carried out by a method of using a gas, a method of using a liquid, etc.

The activation treatment using a gas may be fluorizing. The fluorizing is carried out by putting the cast base formed of the cobalt-chromium based alloy in a furnace for heating treatment, heating the cast base formed of the cobalt-chromium based alloy at 200°C to 500°C in a fluorine based gas atmosphere, keeping the temperature for 10 min to 180 min. Consequently, the chromium oxide on the surface is replaced into chromium fluoride.

The fluorine based gas suitable for the fluorizing may be $NF_3$, $BF_3$, $CF_4$, HF, $SF_6$, $C_2F_6$, $WF_6$, $CHF_3$, $SiF_4$, $ClF_3$, etc. These fluorine based gases may be used alone or in the form of a mixture of 2 or more of them. Generally, these fluorine based gases are used while being diluted with an inert gas such as $N_2$ gas or the like.

The activation treatment using a liquid may be a method of immersion in an acidic solution. As the acidic solution, hydrochloric acid, nitric acid, hydrogen peroxide, sulfuric acid, and hydrofluoric acid may be used alone or in the form of a solution obtained by mixing 2 or more of them. Particularly, a mixed solution obtained by mixing hydrochloric acid and nitric acid; hydrochloric acid, nitric acid, and hydrogen peroxide; or hydrochloric acid and hydrogen peroxide is preferable, and can dissolve the passivation film of chromium oxide on the surface within a short time.

After the diffusion treatment, post-treatment may be carried out in accordance with the surface state. The post-treatment may be acid treatment for removing the soot (in the case of carburizing) adhering to the surface, surface polishing such as mirror polishing, etc.

The sliding alloy member for biomedical use obtained by the diffusion hardening treatment may be used preferably as a sliding member of an artificial joint, for example, for an artificial hip joint, an artificial knee joint, an artificial elbow joint, etc. Particularly, in an artificial joint, 2 sliding members which compose the artificial joint are the sliding alloy members formed of cobalt-chromium based alloy, and if the sliding alloy member for biomedical use of the present invention is adopted for at least one of the sliding alloy members formed of cobalt-chromium based alloy (e.g., a head and/or a stem), the effect of the present invention is sufficiently provided and therefore, it is preferable.

## EXAMPLES

Below, by way of examples, the present invention will be more specifically described. However, the present invention is not limited by the following examples. It is naturally understood that modifications may be properly made and practiced within the scope adaptable to the gists described above and below. All of these are included in the technical scope of the present invention.

11 types of cast bases formed of cobalt-chromium based alloys shown in Table 3 (rod-like materials with diameter: 15 mm and length: 150 mm) were produced. The N amount and C amount of each cast material was controlled by nitrogen partial pressure and graphite addition amount at the time of dissolution. After the casting, each obtained rod-like material was cut into disk-like form with a thickness of about 2 mm, and wet polishing was carried out using SiC paper to obtain each cast base formed of a cobalt-chromium based alloy.

The N (nitrogen) amount of each cast base formed of a cobalt-chromium based alloy obtained in the above-mentioned manner was measured by an inert gas fusion method. The C (carbon) amount was measured by an infrared absorption method after combustion, Si amount was measured by absorptiometry, and the contents of other components shown in Table 3 were measured by ICP spectrometry. To confirm the average crystal grain diameter (the average value of equivalent circular diameter of crystal grains in one arbitrary visual field) of each cast base formed of the cobalt-chromium based alloy, after the observation face was mirror-finished by wet polishing, etching was carried out in an acidic solution, and macro-structure observation was carried out. Fig. 8 shows a representative macro-structure photograph (macro-structure photograph of No. 7 in Table 3). It was confirmed that all of the produced cast bases had an average crystal grain diameter of 1000 μm or larger as shown in Fig. 8.

(Measurement of volume fraction of fcc phase of cast base formed of cobalt-chromium based alloy (before carburizing))

Using the bases, the volume fraction of the fcc phase was measured by X-ray diffraction. That is, using an X-ray diffraction apparatus (RINT 1500, manufactured by Rigaku Corporation), the measurement was carried out in a range of 2θ = 45° to 53° in the condition: a target: Cu, a target output: 40 kV-200 mA, slit system: light reception: 0.3 mm, vertical length: 2 mm, sampling step: 0.02°, measurement time: 8 sec/step while rotating and swinging each sample in the condition: rotation speed: 60 rpm, swinging angle/cycle:-45°to 45°/4 sec. Among the obtained diffraction peaks, the integrated intensity of each peak expressed in the following calculation formula (1) was measured, and the volume fraction Vγ of the fcc phase (γ-phase) was calculated according to the following calculation formula (1). The volume fraction (volume%) of the fcc phase of each base is shown in Table 3.

[Math 1]

$$V_{\gamma} = 1 - 1 / \left\{ \left( \frac{I_{(200)\gamma}}{I_{(10\bar{1}1)\varepsilon}} \right) \times 1.713 + 1 \right\} \cdots (1)$$

(Fomura1, wherein $I_{(1011)\varepsilon}$ indicates integrated intensity of peak derived from $(1011)_{\varepsilon}$ of ε phase, $I_{(200)\gamma}$ indicates integrated intensity of peak derived from 200) of y phase.)

[Table 3]

| No. | Chemical component composition of cast base formed of cobalt-chromium based alloy (unit: mass %) | | | | | | | | | Fcc phase volume% |
|---|---|---|---|---|---|---|---|---|---|---|
| | Cr | Mo | Ni | Fe | C | N | Si | Mn | Co | |
| 1 | 28.54 | 6.07 | 0.02 | 0.05 | 0.01 | 0.014 | 0.40 | 0.31 | bal. | 13.4 |
| 2 | 29.59 | 6.05 | 0.03 | 0.07 | 0.28 | 0.015 | 0.39 | 0.30 | bal. | 23.1 |
| 3 | 28.05 | 6.06 | 0.32 | 0.15 | 0.20 | 0.039 | 0.92 | 0.55 | bal. | 26.8 |
| 4 | 28.90 | 5.72 | 0.30 | 0.38 | 0.24 | 0.085 | 0.99 | 0.66 | bal. | 46.2 |
| 5 | 29.50 | 5.76 | 0.04 | 0.07 | 0.23 | 0.16 | 0.91 | 0.95 | bal. | 60.2 |
| 6 | 29.60 | 5.69 | 0.04 | 0.06 | 0.33 | 0.20 | 0.85 | 0.93 | bal. | 72.6 |
| 7 | 28.35 | 5.87 | 0.03 | 0.07 | 0.28 | 0.21 | 0.82 | 0.90 | bal. | 76.3 |
| 8 | 28.99 | 6.15 | 0.01 | 0.07 | 0.13 | 0.21 | 0.96 | 0.94 | bal. | 80.4 |
| 9 | 29.32 | 6.25 | 0.01 | 0.04 | 0.004 | 0.22 | 0.99 | 0.99 | bal. | 69.1 |
| 10 | 28.94 | 5.95 | 0.01 | 0.07 | 0.23 | 0.23 | 0.89 | 0.93 | bal. | 73.3 |
| 11 | 29.03 | 5.92 | 0.02 | 0.29 | 0.21 | 0.26 | 0.89 | 0.92 | bal. | 81.4 |

Next, the activation treatment and carburizing were successively carried out for the above-mentioned bases. Concretely, after subjected to activation treatment using a fluorine based gas (by keeping at 350°C for 2 hours in $NF_3$ gas atmosphere), each base was subjected to gas carburizing (by keeping at 500°C for 32 hours in CO + $H_2$ mixture gas atmosphere).

(Unevenness of in-plane hardness)

Vickers hardness was measured at a plurality of points in a single plane of each sample after the carburizing. The measurement was carried out at a load of 50 gf with a Vickers hardness measurement apparatus.

As a result, Nos. 1, 3 and 4 showed non-uniform hardness in a single plane as same as the above-mentioned No. 2, and on the other hand, Nos. 5, 6, and 8 to 11 showed almost constant hardness in a single plane as same as the above-mentioned No. 7, regardless of the measurement points.

(Dispersion of carbon profile in carburized layer)

To confirm whether a uniform hardened layer was stably formed by the above-mentioned carburizing or not, the carbon concentration distribution of each sample after the carburizing was measured. Concretely, the measurement was carried out by glow discharge optical emission spectroscopy (GDS). Using JY5000RF-PSS model GDS apparatus manufactured by Jobin Ybon S.A.S. for the glow discharge optical emission spectrometry, the measurement was carried out at low voltage mode (40 W) in vacuum of Ar pressure of 775 Pa.

Fig. 9 shows the result (i.e. the carbon concentration distribution of the carburized layer) separately for examples (Comparative Examples) with the N amount of less than 0.1 mass% and the fcc phase ratio of lower than 50% in the region (region A) and examples (Examples of the present invention) with the N amount of 0.1 mass% or higher and the fcc phase ratio of 50% or higher in the region (region B) shown in Fig. 6.

From Fig. 9, it can be understood that all cast bases formed of cobalt-chromium based alloys show high profile of carbon concentration up to about 20 μm from the surface. Next, if the profiles are compared more in detail, the profiles of Nos. 1 to 4 in the region A were in a significantly wide range of dispersion among the samples. It was supposed that the hardness was uneven depending on the measurement points even in a single plane of one sample and accordingly the hardness was in a significantly wide range of dispersion among the samples. On the other hand, regardless of the component compositions, Nos. 5 to 11 in the region B were found having almost same carbon profile near a surface layer and thus being in uniform solid-solution distribution state.

## Claims

1. A cast base for biomedical use formed of a cobalt-chromium based alloy and having excellent in diffusion hardening treatability, containing 0.1 mass% or more of nitrogen (N), and having volume fraction of an fcc (face centered cubic lattice) phase in the metallic structure of 50% or higher.

2. The cast base for biomedical use formed of the cobalt-chromium based alloy as described in claim 1, wherein the N amount of the cobalt-chromium based alloy is 0.25 mass% in the upper limit and the contents of elements other than N are within ranges standardized in ASTM F75-07.

3. The cast base for biomedical use formed of the cobalt-chromium based alloy as described in claim 1 or 2, having an average crystal grain size in the metallic structure of 1000 μm or greater.

4. A sliding alloy member for biomedical use obtained by diffusion hardening treatment of the cast base formed of the cobalt-chromium based alloy as described in any one of claims 1 to 3 in as-cast state.

5. The sliding alloy member for biomedical use as described in claim 4, wherein the diffusion hardening treatment is carburizing.

6. An artificial joint composed of 2 sliding members forming a sliding face made of a cobalt-chromium based alloy sliding members, wherein at least one of the cobalt-chromium based alloy sliding members is the sliding alloy member for biomedical use as described in claim 4 or 5.

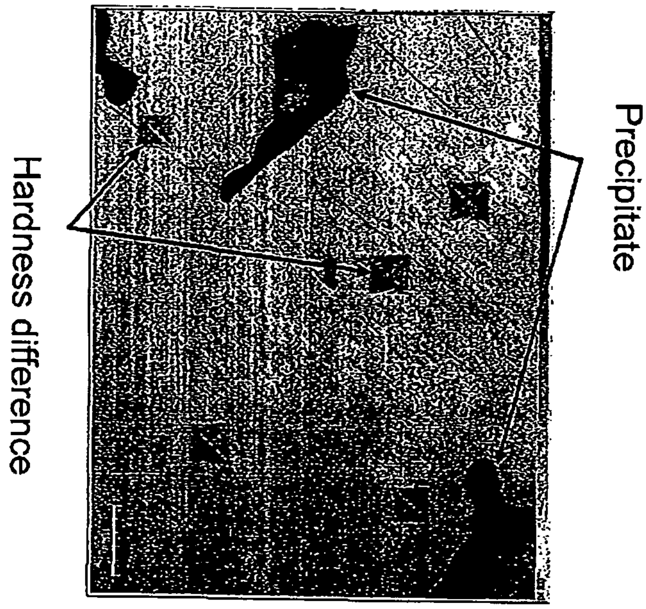


FIG.1

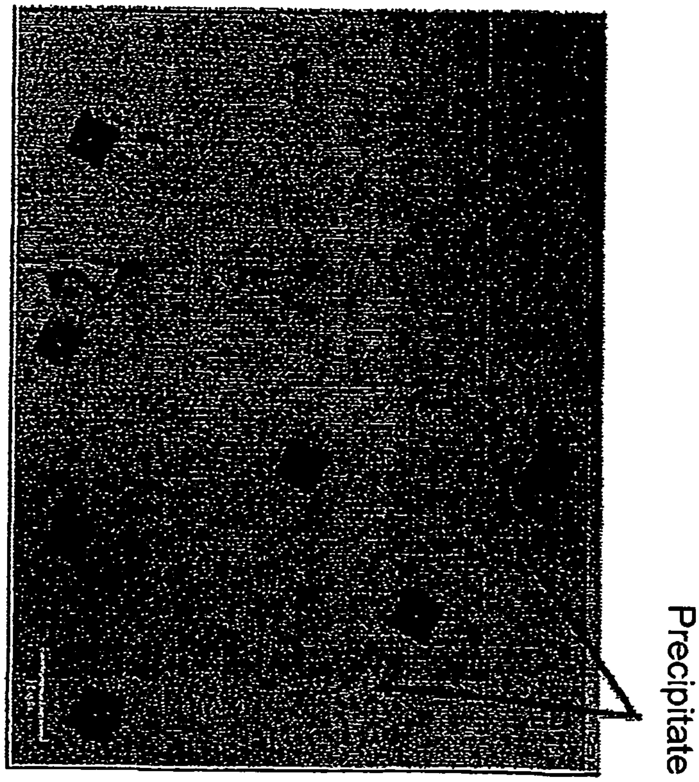


FIG.2

FIG.3

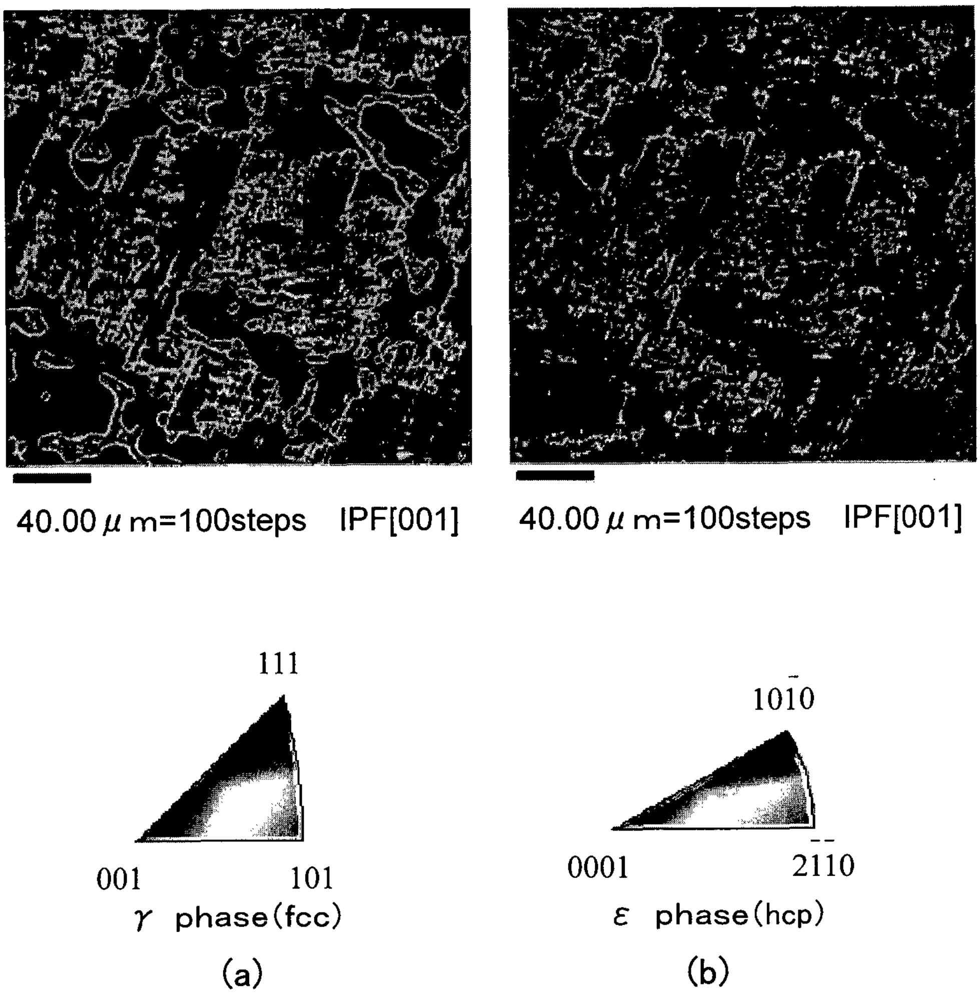
40.00μm=100steps IPF[001]
40.00μm=100steps IPF[001]
111
001
101
γ phase(fcc)
(a)
10$\bar{1}$0
0001
$\bar{2}\bar{1}$10
ε phase(hcp)
(b)

FIG.4

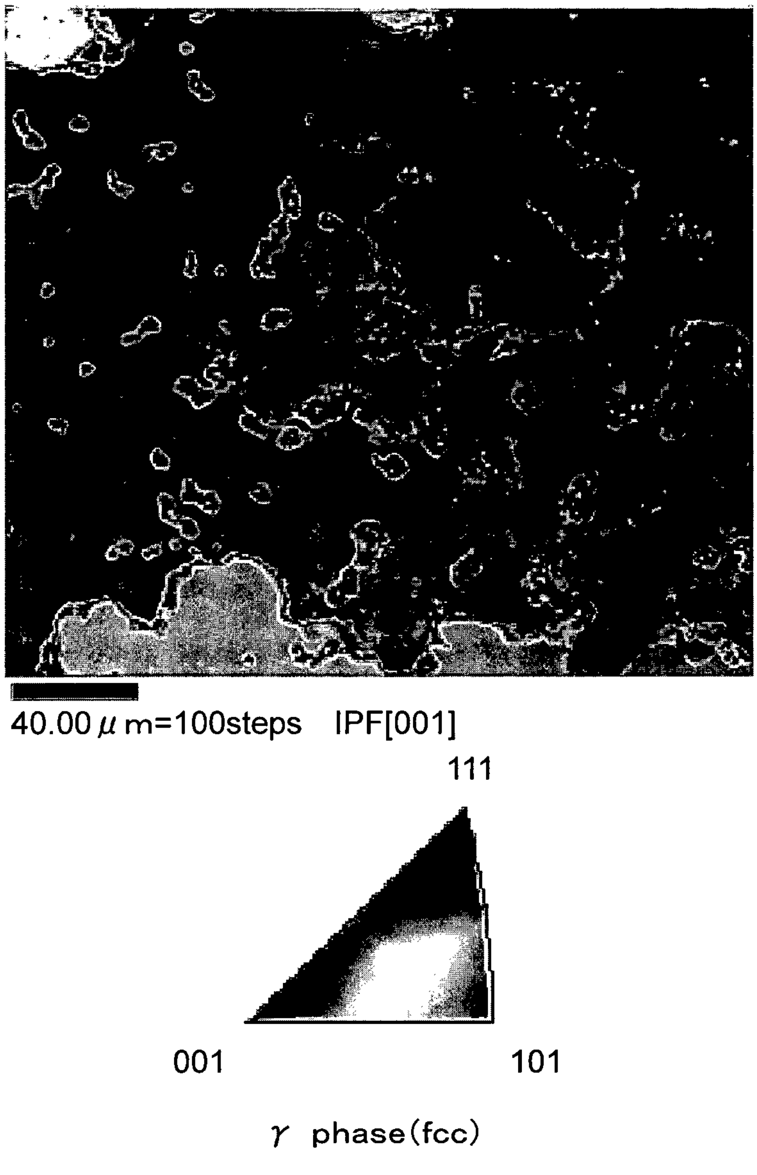

FIG.5

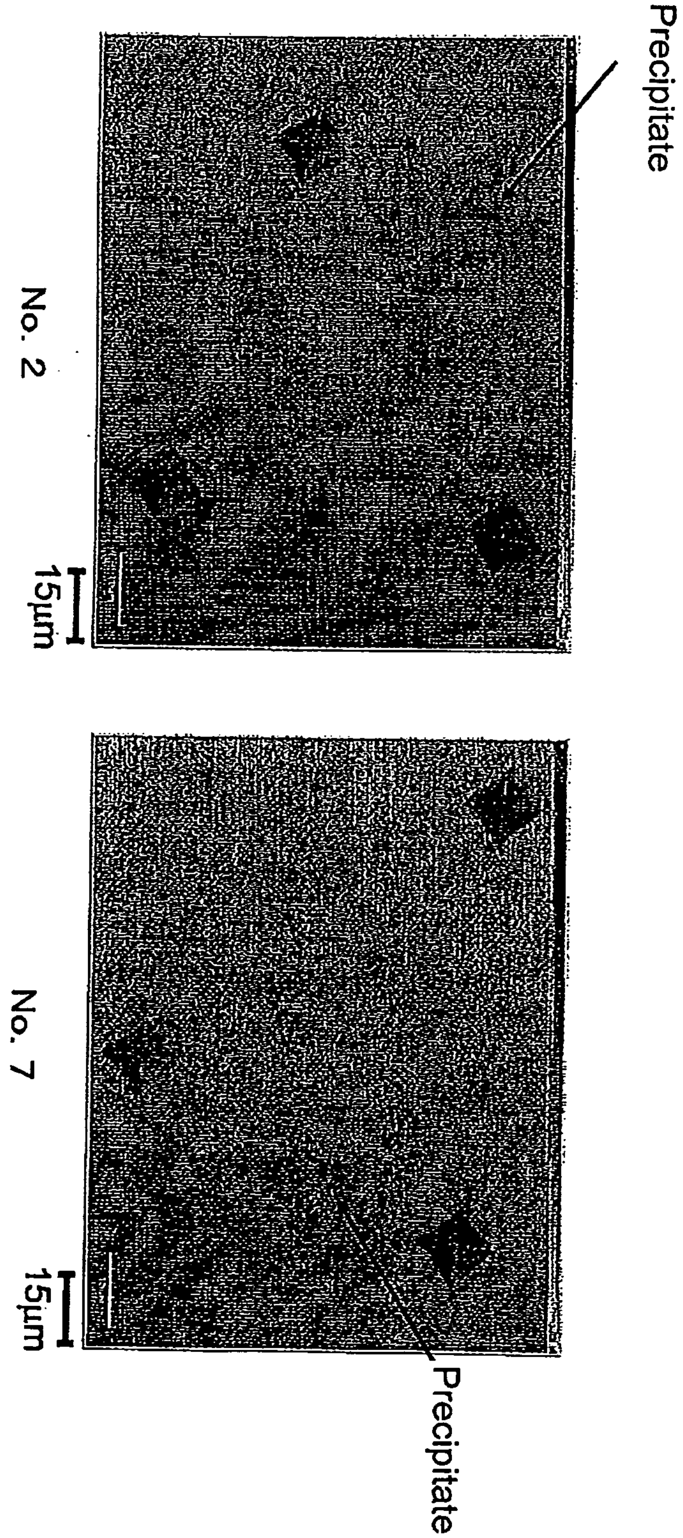

FIG.6

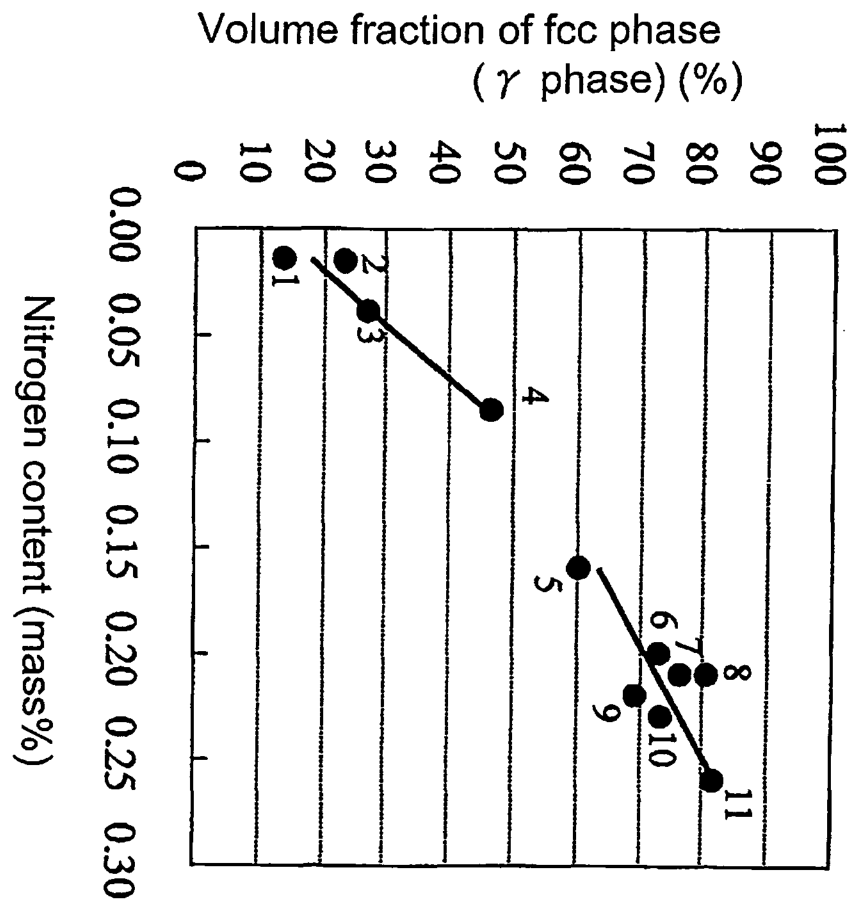
Volume fraction of fcc phase
(γ phase) (%)
100
90
80
70
60
50
40
30
20
10
0
0.00
0.05
0.10
0.15
0.20
0.25
0.30
Nitrogen content (mass%)
1
2
3
4
5
6
7
8
9
10
11

FIG.7

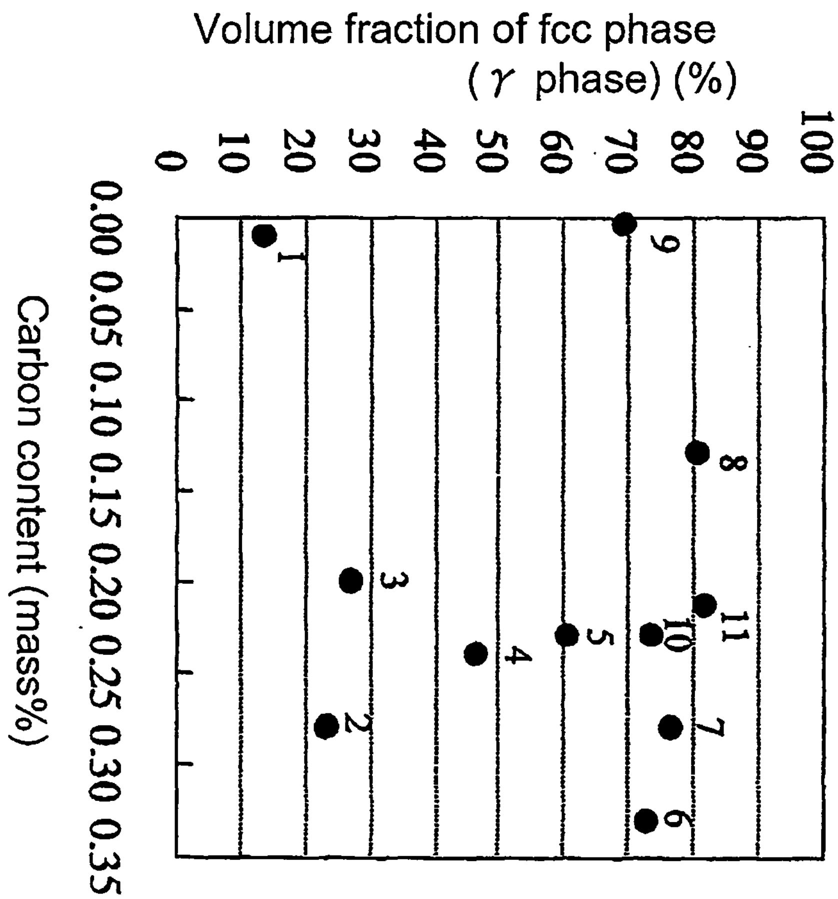
Volume fraction of fcc phase
(γ phase) (%)
100
90
80
70
60
50
40
30
20
10
0
0.00 0.05 0.10 0.15 0.20 0.25 0.30 0.35
Carbon content (mass%)
1
2
3
4
5
6
7
8
9
10
11

FIG.8

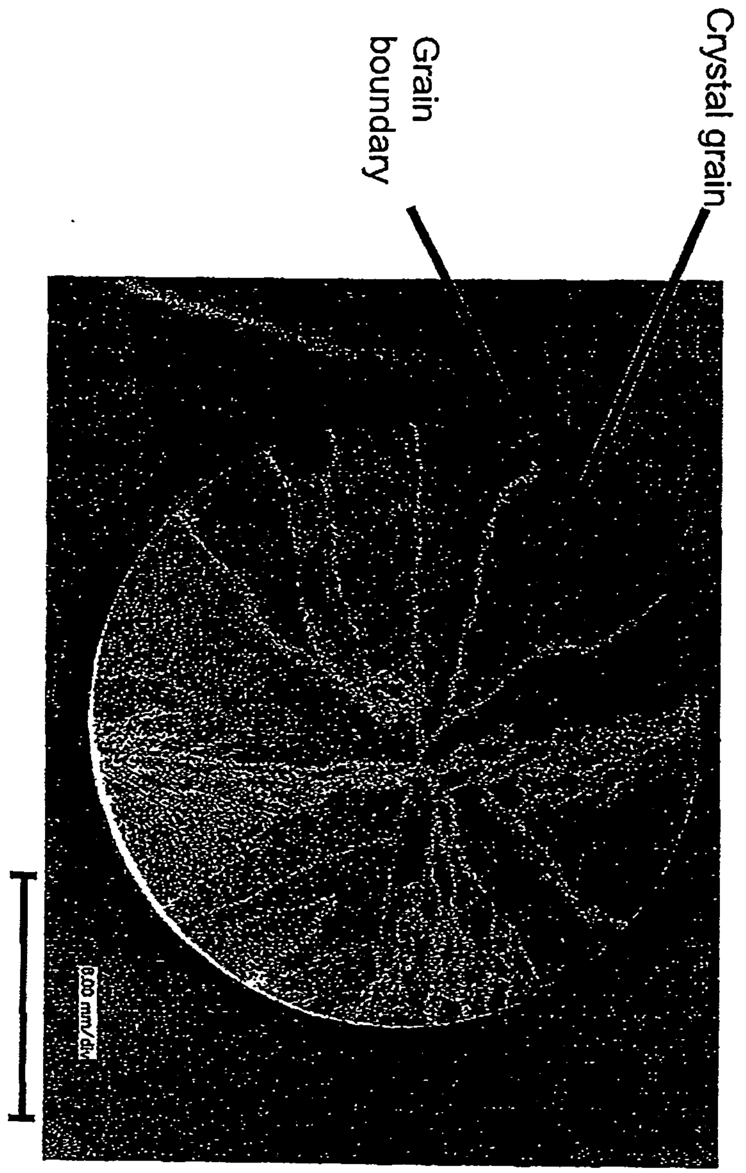

FIG.9

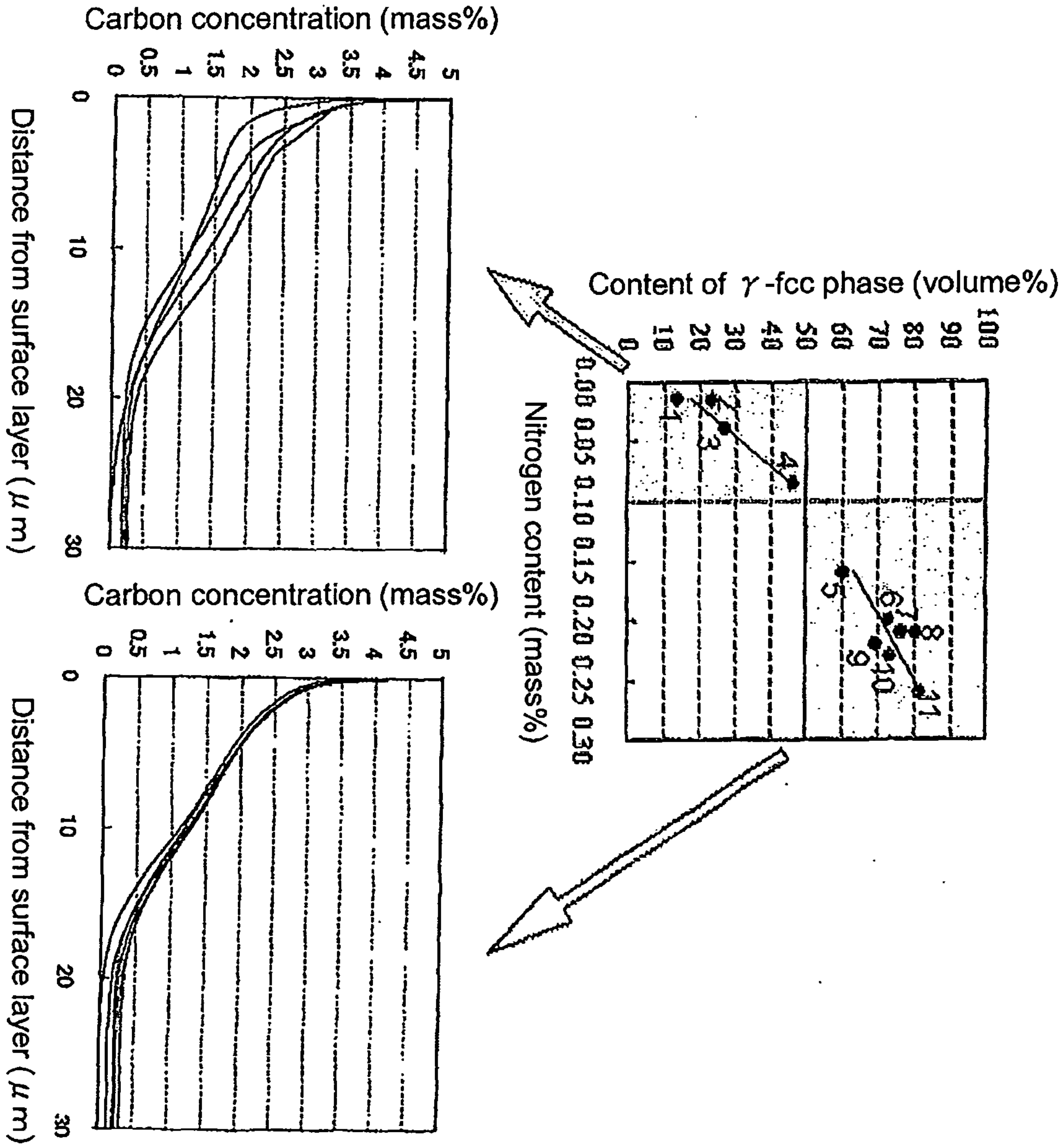
Content of γ-fcc phase (volume%)
0 10 20 30 40 50 60 70 80 90 100
0.00 0.05 0.10 0.15 0.20 0.25 0.30
Nitrogen content (mass%)
1 2 3 4 5 6 7 8 9 10 11
Carbon concentration (mass%)
0 0.5 1 1.5 2 2.5 3 3.5 4 4.5 5
0 10 20 30
Distance from surface layer (μm)
Carbon concentration (mass%)
0 0.5 1 1.5 2 2.5 3 3.5 4 4.5 5
0 10 20 30
Distance from surface layer (μm)

**INTERNATIONAL SEARCH REPORT**

International application No.
PCT/JP2010/059946

A. CLASSIFICATION OF SUBJECT MATTER
*C22C19/07*(2006.01)i, *A61F2/30*(2006.01)i

According to International Patent Classification (IPC) or to both national classification and IPC

B. FIELDS SEARCHED

Minimum documentation searched (classification system followed by classification symbols)
C22C1/00-49/14, A61F2/30

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| E,X | JP 2010-144184 A (Japan Medical Materials Corp., Kobe Steel, Ltd.), 01 July 2010 (01.07.2010), claims (Family: none) | 1-6 |
| X | JP 2007-277710 A (Japan Medical Materials Corp.), 25 October 2007 (25.10.2007), claims; paragraphs [0020], [0031] (Family: none) | 1-6 |
| X | JP 2009-46760 A (Kobe Steel, Ltd.), 05 March 2009 (05.03.2009), claims; paragraph [0020]; table 1 (Family: none) | 1,2,6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>30 August, 2010 (30.08.10) | Date of mailing of the international search report<br>07 September, 2010 (07.09.10) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2010/059946

C (Continuation). DOCUMENTS CONSIDERED TO BE RELEVANT

| Category* | Citation of document, with indication, where appropriate, of the relevant passages | Relevant to claim No. |
|---|---|---|
| X | JP 2009-46758 A (Kobe Steel, Ltd.), 05 March 2009 (05.03.2009), claims; paragraphs [0022], [0025]; tables 1, 2 (Family: none) | 1,2,6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 54010224 A
- JP 2008111177 A
- JP 2005524772 A
- JP 2007277710 A
- JP 3471041 B